# EUROPEAN PATENT SPECIFICATION

(11) **EP 4 171 452 B1**
(45) Date of publication and mention of the grant of the patent: **31.07.2024**
(21) Application number: 21756030.9
(22) Date of filing: 28.06.2021
(51) Int. Cl.: A61F 5/02, A61F 5/30

(54) **ORTHOPAEDIC CORSET**
ORTHOPÄDISCHES KORSETT
CORSET ORTHOPÉDIQUE

(30) Priority: 29.06.2020 IT 202000015673
(43) Date of publication of application: 03.05.2023
(73) Proprietor: Giontella, Massimo, 50123 Firenze (FI) (IT)
(72) Inventor: Giontella, Massimo, 50123 Firenze (FI) (IT)
(74) Representative: Firmati, Leonardo
(86) International application number: PCT/IB2021/055751
(87) International publication number: WO 2022/003527

(56) References cited:
- EP-A1- 1 902 691
- WO-A1-2013/123246
- DE-A1- 10 329 454
- US-A- 3 282 264
- US-A- 5 295 947
- US-A1- 2016 206 467
- CURBELL: "2019 Curbell Plastics", 31 December 2019 (2019-12-31), pages 1 - 1, XP055784392, Retrieved from the Internet <URL:https://www.curbellplastics.com/Research-Solutions/Technical-Resources/Technical-Resources/KYDEX-Data-Sheet> [retrieved on 20210310]

## Description

### Technical field

The present invention relates to an orthopaedic corset.

In particular, the present invention concerns an orthopaedic corset intended for the treatment of pathologies inherent to the spinal column.

### Background art

The spinal column, also known as the spine, is comprised of a coordinated series of segments, the vertebrae, separated in the front part by cartilaginous structures known as intervertebral discs.

Anatomically, the spine is comprised by a cervical tract which comprises seven vertebrae (from C1 to C7), a thoracic (or dorsal) tract which comprises twelve vertebrae (from T1 to T12), a lumbar tract which comprises five vertebrae (from L1 to L5), the sacrum comprised of the fusion of five vertebrae forming a single structure (from S1 to S5) and the coccyx. The spine is a complex structure able to guarantee opposition to gravity, thus enabling an erect posture and guaranteeing the balance between forces and resistances, necessary for locomotion and for every other kinetic function.

The vertebrae represent the functional unit of the spinal column.

They are comprised of a front structure called the body and a rear one comprised of arches (peduncle and laminae), having transverse and spinous processes.

The vertebral bodies are connected to one another by means of cartilaginous discs which enable relative movements of the rigid bone structures.

The cartilaginous discs have a central part called the nucleus pulposus comprising a deformable gel, loaded with water, which acts as a shock absorber, surrounded by a peripheral annulus fibrosus which contains it under mechanical strain.

The rear pillar of the vertebrae is represented by the arches and the apophyses which have the role of guiding the movement of the adjacent vertebrae, connected to each other by cartilaginous joints, under the action of paravertebral muscles.

The two fundamental mechanical requirements of the spine are therefore rigidity, necessary for static efficiency and for the protection of the important organs that are inside it (marrow and nerves), and flexibility.

When for arthrosic, traumatic, osteoporotic deformation processes etc. the bone structures, discs and rear joints lose their anatomic structure and functionality, the subject begins to have progressive problems in terms of erect posture and in walking, for the compression of the connected nervous structures. Such types of pathologies, although localised on the entire spine, are actually more frequent in the lumbar region which represents the area where the load stress is concentrated.

To overcome such types of problems, different orthoses have been designed: tutors (made of plastic and metal material) and corsets, mainly made of natural or synthetic fabric, clearly more comfortable, which in the rear lumbar region have a plurality of pockets into which reinforcement splints are inserted (commonly four/five, appropriately distanced from one another) made of metal or plastic material which have the function of supporting the spine by relieving compression on the nervous structures. However, such solutions offer responses to the problems described above which are only partially effective as the splints are only made solid in their function as they are inserted into the fabric which is in actual fact yielding in itself.

Therefore, in substance, the support function of the splints is largely exercised by the splints themselves individually, without such action actually being shared among all the splints and also without the distribution of the forces at play which are therefore localised, to the detriment of the patient wearing the orthosis.

US patent US3,282,264A is considered to represent the closest prior art, it discloses an orthopaedic corset in accordance with the preamble of claim 1.

Further examples of orthopaedic corsets, which also comprise components made of Kydex (R), are described inWO2013/123246 or US 5,295,947 A.

### Aim of the invention

The aim of the present invention is therefore that of solving the drawbacks of the prior art mentioned above.

In particular, this invention has for an aim to provide an orthopaedic corset which can support the spinal column safely and effectively.

Furthermore, the aim of the present invention is to propose an orthopaedic corset that is versatile i.e. easily adaptable to the specific needs of different patients.

Yet another aim of the invention is to provide an orthopaedic corset which is simple and inexpensive to make and practical to use.

The technical characteristics of the invention, with reference to the above aims, can be easily inferred from the appended claims.

### Brief description of the drawings

The advantages of the invention will become more apparent from the detailed description which follows, with reference to the accompanying drawings which illustrate preferred embodiments of the invention provided merely by way of example without restricting the scope of the inventive concept, and in which:
- Figure 1 is a schematic rear elevation view, with some parts transparent, to better illustrate others, of a first embodiment of the orthopaedic corset according to the invention worn by a user;
- Figure 2 is a schematic rear elevation view, with some parts transparent, to better illustrate others, of a variant of the orthopaedic corset of Figure 1;
- Figure 3 is a schematic front elevation view of a detail of the orthopaedic corset of the previous figures.

### Detailed description of preferred embodiments of the invention

With reference to the accompanying drawings, the numeral 1 denotes in its entirety an orthopaedic corset according to the present invention.

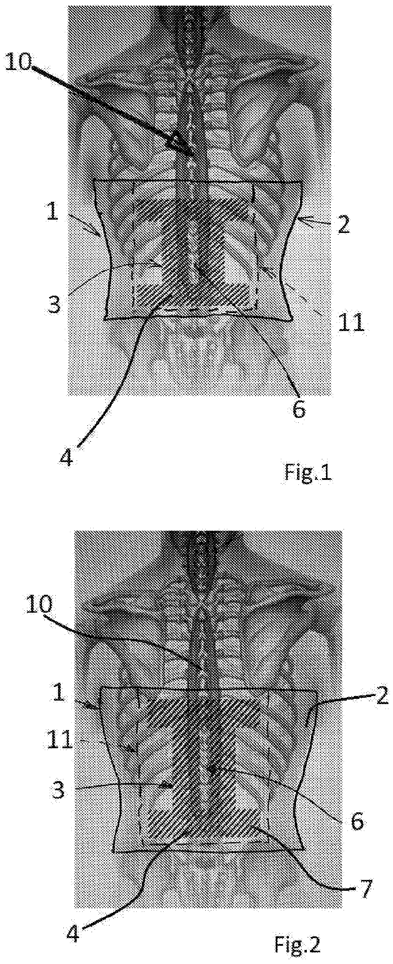

With reference to Figures 1 and 2, the corset 1 comprises a floppy containment structure 2 which is adapted to be arranged stably, and however removably, around the torso of a user or patient.

The floppy containment structure 2 is advantageously made of natural or synthetic fabric.

Preferably, the floppy structure 2 comprises portions, not illustrated in detail in the accompanying drawings, at least partially elastic and adapted to facilitate the positioning of the corset 1 on the patient's torso as well as reaching the right tensioning, as for a normal orthopaedic corset of the known type.

The corset 1 further comprises a rear stiffening means 3 for stiffening the floppy structure 2 constrained to the latter.

The stiffening means 3 comprises a plate 4 made as a single body.

The plate 4 comprises at least one portion with a quadrangular extension 5 and having a central gap 6 with a longitudinal extension configured to be arranged in use overlapping with a portion of the spinal column 10 of the user.

The central gap 6 of the portion with a quadrangular extension 5 enables direct strain on the spinal apophyses of the user's spinal column 10 to be avoided and at the same time optimised aeration of the corset 1 as a whole to be obtained.

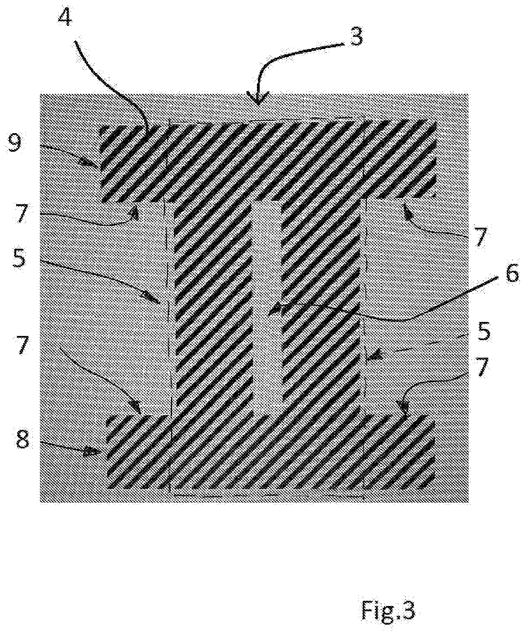

Advantageously the plate 4 comprises a plurality of lateral wings 7 emerging in a projecting way from the portion with a quadrangular extension 5. Preferably, there are four lateral wings 7 arranged at the ends of the plate 4, at a lower base 8 and an upper base 9 of the portion with a quadrangular extension 5, as shown in the accompanying Figures 1 - 3.

Therefore, advantageously, the lateral wings 7 define, in combination with the portion with a quadrangular extension 5 a lower base 8 of the plate 4, adapted in use to be arranged in support on a lower portion of the user's spinal column 10 and an upper base 9 of the plate 4, adapted in use to be arranged in support on an upper portion (with respect to said lower base 8) of the user's spinal column 10.

The floppy structure 2 comprises a pocket 11 configured to contain the plate 4 made as a single body.

The pocket 11 comprises an inlet portion configured to enable the insertion and removal of the plate 4 from the pocket 11 itself.

Preferably, the inlet portion of the pocket 11 comprises reversible closing means (e.g. a zip) for preventing the risk of the plate 4 coming out of the pocket 11 itself.

The pocket 11 is made so as to precisely contain the plate 4 but without enabling it to have substantially any internal movement.

In other words, the compartment defined by the pocket 11 is shaped slightly larger than the dimensions of the plate 4, just to enable the access of the latter but without the plate 4 being able to make any particular movement once inserted into the pocket 11.

The circumstance that the plate 4 is inserted into a pocket of the precisely shaped corset 1 and supported thereby with simple connection and support portions ensures that the plate 4 itself interacts completely integrally with the floppy structure 2.

This enables complete adhesion of the plate 4 to the patient's body and therefore to the spine with consequent direct intervention on the paravertebral muscles.

Advantageously, at the area occupied during use by the central gap 6, the pocket 11 has removable connection means and, not illustrated, adapted to stably join during use, the two internal faces of the pocket 11 itself, thus ensuring even more stable positioning of the plate 4 inside the pocket 11. This contributes to making the plate 4 even more integral to the floppy structure 2.

The mentioned and not illustrated removable connection means advantageously comprise portions of Velcro^{®}, also not illustrated, adapted to enable a rapid connection of the two mentioned faces of the pocket 11 and equally as quick a disengagement thereof, precisely at the central gap 6 of the plate 4.

Advantageously, through the above described peculiar combination of plate 4 and pocket 11 of suitable dimensions, it is possible to support the spine without locking it but also leaving it free in the bending-extension and lateral movements, being opposed to the compression of the joint and nervous structures generated in arthrosic and degenerative lesions.

The fact that the spine is blocked, as takes place in many known corset solutions, has the unpleasant consequence of generating hypotonia and hypotrophia of the paravertebral muscles, therefore when the corset is removed the spinal column yields even more than when the corset was first put on. With the orthopaedic corset 1 according to the invention, on the other hand, the compromised function of the paravertebral muscles is optimised, therefore the benefit continues also after the removal of the corset.

The plate 4 is advantageously made of Kydex^{®}.

Kydex^{®} is a plastic material resulting from the combination of PVC and acrylic, characterised by specific biomechanical reactions that provide optimised solutions in the treatment of the lumbar pathology.

Kydex^{®} is a polyvinyl acrylic-chloride composite designed for thermoforming manufacturing and combines the properties both of the acrylic components and polyvinyl chloride.

Rigidity and formability are obtained from the acrylic whereas tenacity, chemical resistance and an excellent internal finish are obtained from the PVC.

Advantageously the Kydex^{®} used for the present invention is Kydex^{®} 100 whose composition is not divulged by the manufacturer except as a combination of acrylic and PVC, i.e. as a mixture of polyvinyl chloride, chlorinated polyvinyl chloride, acrylic polymer, process aids, impact modifiers, heat stabilisers and lubricants.

The indication of Kydex^{®} 100 however enables a person skilled in the art to perform the invention as it is a specific single material.

Kydex^{®} 100 has the following characteristics:
impact resistance equal to 961 J/m, modulus of elasticity 2310 MPa, tensile strength 42MPa, Rockwell hardness 94 and heat deflection temperature 78.3°C. In particular, the sheet-shaped Kydex^{®} has very different reactions according to its thickness; for example with 1 mm thickness of Kydex^{®} it is more elastic under bending-extensive strain whereas it is more resistant under compressive strain.

With 1.5 mm thickness the bending-extensive elasticity is notably reduced whereas already with 2-3 mm the Kydex^{®} sheet is more rigid and the bending-extension is substantially cancelled out.

Such characteristics enable spinal column 10 pathologies to be treated with plates 4 having different and optimised thicknesses according to the gravity of the user's pathology.

If the lesion is mild, for example, a Kydex^{®} plate 4 having a thickness of 1 mm supports the spine while still leaving maximum motility for the lumbar column 10.

If instead the lesion is more severe, increasing the thickness also increases the support for the spinal column 10 which becomes much more effective and at the same time notably reduces motility.

Experimental tests have shown that in the advantageously selected thicknesses comprised between 1 and 1.5 mm the Kydex^{®} plate 4 shows elastic bending-extensive behaviour and stiffens under compression giving the corset 1 high functionality while leaving the bending-extension movements of the spinal column free.

An embodiment of the orthopaedic corset 1, not illustrated, according to the present invention comprises a plate 4 having portions with different thicknesses.

In this way the possibility of the orthopaedic technician to adapt the characteristics of the corset 1 to the user's actual pathology is optimised. The present invention has important advantages, also already highlighted above, and overcomes the drawbacks of the prior art.

A first important advantage consists of the fact that through the use of a plate made of a single body it is possible to obtain an optimised and structural response in terms of support for the spinal column.

In fact, the single body enables perfect and uniform transmission of loads in order to support the user's spinal column.

A further advantage consists of the fact that an orthopaedic corset according to the present invention enables suitable aeration of the user's skin in contact with the corset itself to be obtained, mainly thanks to the presence of the central gap.

Yet another advantage connected with the use of the corset 1 according to the invention consists of the limited invasiveness thereof in aesthetic terms; in fact, the complete insertion of the plate 4 into the pocket 11, enables complete adhesion of the floppy structure 2 to the patient's body to be obtained, which is even modelled with a great aesthetic improvement.

## Claims

1. Orthopaedic corset (1) for the support of the spinal column (10), comprising:
- a floppy containment structure (2) adapted to be arranged stably around the torso of a user,
- rear stiffening means (3) for stiffening said floppy structure (2), constrained to the latter, said rear stiffening means (3) comprising a plate (4) made of a single body, comprising at least one portion with a quadrangular extension (5) and having a central gap (6) with a longitudinal extension configured to be arranged overlapping with a portion of the user's spinal column (10), said floppy structure (2) comprising a pocket (11) configured to contain said plate (4) made of a single body, **characterized in that** said plate (4) is made of Kydex^{®}, said Kydex^{®} being a polyvinyl acrylic chloride composite, and having a thickness comprised between 1 mm and 1.5 mm.

2. Orthopaedic corset (1) according to claim 1, **characterised in that** said plate (4) comprises a plurality of lateral wings (7) emerging in a projecting way from said portion with a quadrangular extension (5).

3. Orthopaedic corset (1) according to claim 2, **characterised in that** said lateral wings (7) define, in combination with said portion with a quadrangular extension (5), a lower base (8) of said plate (4), adapted to be arranged in support on a portion of the user's spinal column (10).

4. Orthopaedic corset (1) according to any one of the preceding claims, wherein said pocket (11) comprises an inlet portion configured to allow the insertion and removal of said plate (4) from said pocket (11) itself.

5. Orthopaedic corset (1) according to claim 4, wherein said pocket (11) comprises removable connection means for connecting two inner faces arranged at an area occupied in use by said central gap (6) of said plate (4), once inserted into said pocket (11).

6. Orthopaedic corset (1) according to claim 5, wherein said removable connection means comprise portions of Velcro^{®}.

7. Orthopaedic corset (1) according to any one of the preceding claims, wherein said pocket (11) is configured to precisely house said plate (4), so as to limit or reduce the movement of the plate (4) within the pocket (11) itself.

8. Orthopaedic corset (1) according to any one of the preceding claims wherein said plate (4) has portions of different thicknesses.

## Patentansprüche

1. Orthopädisches Korsett (1) für die Abstützung der Wirbelsäule (10), umfassend:
- eine biegsame Aufnahmestruktur (2), die geeignet ist, stabil um den Rumpf eines Benutzers herum angeordnet zu werden,
- hintere Versteifungsmittel (3) zum Versteifen der biegsamen Struktur (2), die an letztere gebunden sind, wobei die hinteren Versteifungsmittel (3) eine Platte (4) umfassen, die aus einem einzigen Körper ausgebildet ist, umfassend mindestens einen Abschnitt mit einer viereckigen Ausdehnung (5) und aufweisend einen zentralen Spalt (6) mit einer Längsausdehnung, die ausgelegt ist, um mit einem Abschnitt der Wirbelsäule (10) des Benutzers überlappend angeordnet zu werden, wobei die biegsame Struktur (2) eine Tasche (11) umfasst, die ausgelegt ist, um die Platte (4), die aus einem einzigen Körper ausgebildet ist, aufzunehmen, **dadurch gekennzeichnet, dass** die Platte (4) aus Kydex^{®} hergestellt ist, wobei der Kydex^{®} ein Polyvinylacrylchlorid-Verbundstoff ist und eine Dicke zwischen 1 mm und 1,5 mm aufweist.

2. Orthopädisches Korsett (1) nach Anspruch 1, **dadurch gekennzeichnet, dass** die Platte (4) eine Vielzahl von seitlichen Flügeln (7) umfasst, die aus dem Abschnitt mit einer viereckigen Ausdehnung (5) hervorstehend heraustreten.

3. Orthopädisches Korsett (1) nach Anspruch 2, **dadurch gekennzeichnet, dass** die seitlichen Flügel (7) in Kombination mit dem Abschnitt mit einer viereckigen Ausdehnung (5) eine untere Basis (8) der Platte (4) definieren, die geeignet ist, zur Abstützung an einem Abschnitt der Wirbelsäule (10) des Benutzers angeordnet zu werden.

4. Orthopädisches Korsett (1) nach einem der vorhergehenden Ansprüche, wobei die Tasche (11) einen Einlassabschnitt umfasst, der so ausgelegt ist, dass er das Einführen und Entfernen der Platte (4) aus der Tasche (11) selbst ermöglicht.

5. Orthopädisches Korsett (1) nach Anspruch 4, wobei die Tasche (11) entfernbare Verbindungsmittel zum Verbinden von zwei Innenflächen umfasst, die an einem Bereich angeordnet sind, der im Gebrauch von dem zentralen Spalt (6) der Platte (4) eingenommen wird, sobald sie in die Tasche (11) eingeführt werden.

6. Orthopädisches Korsett (1) nach Anspruch 5, wobei die entfernbaren Verbindungsmittel Abschnitte aus Velcro^{®} umfassen.

7. Orthopädisches Korsett (1) nach einem der vorhergehenden Ansprüche, wobei die Tasche (11) so ausgelegt ist, dass sie die Platte (4) genau unterbringt, um die Bewegung der Platte (4) innerhalb der Tasche (11) selbst zu begrenzen oder zu reduzieren.

8. Orthopädisches Korsett (1) nach einem der vorhergehenden Ansprüche, wobei die Platte (4) Abschnitte unterschiedlicher Dicke aufweist.

## Revendications

1. Corset orthopédique (1) pour le soutien de la colonne vertébrale (10), comprenant :
- une structure de contention souple (2) adaptée pour être disposée de manière stable autour du torse d'un utilisateur,
- des moyens de raidissement arrière (3) pour raidir ladite structure souple (2), solidaires de cette dernière, lesdits moyens de raidissement arrière (3) comprenant une plaque (4) faite d'un seul corps, comprenant au moins une partie avec une extension quadrangulaire (5) et comportant un espacement central (6) doté d'une extension longitudinale configurée pour être disposée en se chevauchant avec une partie de la colonne vertébrale (10) de l'utilisateur, ladite structure souple (2) comprenant une poche (11) configurée pour contenir ladite plaque (4) constituée d'un seul corps, **caractérisée en ce que** ladite plaque (4) est constituée de Kydex^{®}, ledit Kydex^{®} étant un composite de chlorure de polyvinyle et d'acrylique, et ayant une épaisseur comprise entre 1 et 1,5 mm.

2. Corset orthopédique (1) selon la revendication 1, **caractérisé en ce que** ladite plaque (4) comprend une pluralité d'ailes latérales (7) émergeant en saillie de ladite partie à extension quadrangulaire (5).

3. Corset orthopédique (1) selon la revendication 2, **caractérisé en ce que** lesdites ailes latérales (7) définissent, en combinaison avec ladite partie à extension quadrangulaire (5), une base inférieure (8) de ladite plaque (4), adaptée pour être disposée en appui sur une partie de la colonne vertébrale (10) de l'utilisateur.

4. Corset orthopédique (1) selon l'une quelconque des revendications précédentes, dans lequel ladite poche (11) comprend une partie d'entrée configurée pour permettre l'insertion et le retrait de ladite plaque (4) de ladite poche (11) elle-même.

5. Corset orthopédique (1) selon la revendication 4, dans lequel ladite poche (11) comprend des moyens de raccordement amovibles pour relier deux faces intérieures disposées en correspondance d'une zone occupée, lors de l'utilisation, par ledit espacement central (6) de ladite plaque (4), une fois insérée dans ladite poche (11).

6. Corset orthopédique (1) selon la revendication 5, dans lequel lesdits moyens de raccordement amovibles comprennent des parties de Velcro^{®}.

7. Corset orthopédique (1) selon l'une quelconque des revendications précédentes, dans lequel ladite poche (11) est configurée pour loger précisément ladite plaque (4), de manière à limiter ou à réduire le mouvement de la plaque (4) à l'intérieur de la poche (11) elle-même.

8. Corset orthopédique (1) selon l'une quelconque des revendications précédentes, dans lequel ladite plaque (4) comporte des parties d'épaisseurs différentes.
